# EUROPÄISCHE PATENTSCHRIFT

(11) **EP 2 180 002 B1**
(45) Veröffentlichungstag und Bekanntmachung des Hinweises auf die Patenterteilung: **26.12.2012**
(21) Anmeldenummer: 09014638.2
(22) Anmeldetag: 18.11.2005
(51) Int. Cl.: C07F 9/30, C07D 213/20, C07D 233/54, C07F 9/11, C07F 9/02, C07F 9/09, C07F 9/32, C07F 9/40

(54) **Verfahren zur Herstellung von Onium-Salzen mit Diakylphosphinat Anionen mit geringem Halogenid-Gehalt**
Method for producing onium salts with dialkyl phosphonate anions with low halogenide content
Procédé de fabrication de sels d'onium avec des anions de diakylphosphinate à teneur moindre en halogénides

(30) Priorität: 14.12.2004 DE 102004060075
(43) Veröffentlichungstag der Anmeldung: 28.04.2010
(62) Teilanmeldung aus: 05815439.4
(73) Patentinhaber: Merck Patent GmbH, 64293 Darmstadt (DE)
(72) Erfinder: Ignatyev (Mykola), Nikolai, Dr., 47058 Duisburg (DE); Welz-Biermann, Urs, Prof.Dr., Liaoning Province Dalian 116021 (CN); Kucheryna, Andriy, Dr., Kyiv 4215 (UA); Willner, Helge, Prof.Dr., 45481 Muelheim/Ruhr (DE)

(56) Entgegenhaltungen:
- WO-A2-2004/106288
- DATABASE CA [Online] CHEMICAL ABSTRACTS SERVICE, COLUMBUS, OHIO, US; 1990, KOVALEVA, T. V. ET AL: "Perfluoroalkylphosphonic acids and their derivatives" XP002568403 gefunden im STN Database accession no. 1990:235432 & ZHURNAL OBSHCHEI KHIMII , 59(11), 2512-15 CODEN: ZOKHA4; ISSN: 0044-460X, 1989,
- KOVALEVA T V ET AL: "Perfluoroalkyl- phosphonic acids and their derivatives", JOURNAL OF GENERAL CHEMISTRY USSR, CONSULTANTS BUREAU, NEW YORK, NY, US, vol. 59, no. 11, 20 April 1990 (1990-04-20), pages 2245-2248, XP002246155, ISSN: 0022-1279

## Beschreibung

Die Erfindung betrifft ein Verfahren zur Herstellung von Onium-Salzen mit Dialkylphosphinat Anionen durch Umsetzung eines Onium-Halogenids mit einem Dialkylphosphinsäurealkylester oder einem Trialkylsilylester oder einem gemischten Alkyl-trialkylsilylester der Dialkylphosphinsäure nach Anspruch 1.

Eine große Anzahl von Onium-Salzen, auch Dialkylphosphinate, sind als ionische Flüssigkeiten verwendbar. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄⁻, PF₆⁻, SbF₆⁻, NO₃⁻, CF₃SO₃⁻, (CF₃SO₂)₂N⁻, ArylSO₃⁻, CF₃CO₂⁻, CH₃CO₂⁻ oder Al₂Cl₇⁻ zu nennen.

Eine generelle Methode zur Herstellung von Onium-Dialkylphosphinaten ist durch Jean, Bull. Soc. Chim. Fr. (1957), 783-785 oder R. Jentzsch et al, J. Prakt. Chem. (1977), 319, 871-874 bekannt.
Ein Nachteil dieser Methode ist jedoch, dass ein Substituent des entstehenden Onium-Kations immer der entsprechenden Alkylgruppe des Alkylesters entspricht. Erwünscht sind jedoch unsymmetrisch substituierte Onium-Salze, d.h. Salze, bei denen die Alkylgruppe des eingesetzten Esters nicht Substituent des entstehenden Onium-Salzes wird.

Unsymmetrische Onium-Salze mit Dialkylphosphinat-Anionen, wie zuvor definiert, können auch durch eine Metathese hergestellt werden, indem ein Oniumhalogenid mit einem entsprechenden Alkalimetallsalz der entsprechenden Säure umgesetzt würde. Das anfallende Alkalimetallhalogenid, z.B. Natriumchlorid, muss jedoch durch eine zusätzliche Aufreinigungsmethode entfernt werden. Die Verunreinigung durch Halogenid-lonen, beispielsweise Chlorid-lonen, größer als 1000 ppm (0,1 %), reduziert die Anwendbarkeit der ionischen Flüssigkeit, insbesondere in der Anwendung für elektrochemische Prozesse. Daher ist bei Verfahren zur Herstellung von Onium-Salzen, insbesondere ionischer Flüssigkeiten, die Technologie von entscheidender Bedeutung, damit diese durch die Reaktion per se oder die Reaktionsführung mit geringen Verunreinigungen synthetisiert werden können und so weitere kostenintensive zusätzliche Verfahrensschritte bei der Synthese entfallen.

WO 2004/106288 beschreibt ein Verfahren zur Herstellung von Guanidinium-Salzen mit den Anionen ausgewählt aus der Gruppe bestehend aus:
[(FSO₂)₂N]⁻, [R^{F}CF₂SO₃]⁻, [(R^{F'}SO₂)₂N]⁻, [(R^{F'}SO₂)₃C]⁻, [(FSO₂)₃C]⁻, [R^{F}CF₂C(O)O]⁻, [P(CₙF₂ₙ₊₁₋ₘHₘ)_{y}F_{6-y}]⁻, [P(C₆F₅)_{y}F_{6-y}]-, [R^{F}2P(O)O]⁻, [R^{F}P(O)O₂]²⁻, [BF_{z}R^{F}_{4-z}]⁻, [BF_{z}(CN)_{4-z}]⁻, [N(CF₃)₂]⁻, [N(CN)₂]⁻, [C(CN)₃]⁻ oder 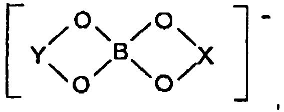 dadurch gekennzeichnet, dass ein Guanidin oder ein Guanidiniumsalz C(NR₂)₃⁺ X⁻, wobei X⁻ ausgewählt ist aus der Gruppe
   F⁻, Cl⁻, Br⁻, I⁻, [HF_{2]}⁻, [CN]⁻, [SCN]⁻, [CH₃COO]⁻, [CF₃COO]⁻, [CF₃SO₃]⁻, [CH₃OSO₃]⁻, [SiFe]²⁻, [BF₄]⁻, [SO₄]²⁻, [NO₃]⁻, Tosylate, Malonate, substituierte Malonate und [CO₃]⁻,
   mit einem Ester AR umgesetzt wird, wobei A aus der Gruppe der zuvor angegebenen Gruppe der Anionen gewählt werden kann und die Reste R wie in Anspruch 1 der WO 2004/106288 definiert sind, wobei jedoch die Subsitutenten R perhalogenierte Alkylgruppen mit 1-20 C-Atomen, Cycloalkylgruppen mit 3-7 C-Atomen, Alkenylgruppen mit 2-20 C-Atomen und Alkinylgruppen mit 2-20 C-Atomen ausgeschlossen sind.

Aufgabe der vorliegenden Erfindung war dementsprechend ein alternatives Verfahren zur Herstellung von Onium-Salzen mit Dialkylphosphinat-Anionen mit geringem Halogenid-Gehalt zur Verfügung zu stellen, welches zu Salzen, vorzugsweise zu unsymmetrisch substituierten Onium-Salzen, mit hoher Reinheit in guter Ausbeute führt und auch für eine großtechnische Produktion geeignet ist.

Selbstverständlich ist ein solches Verfahren dann auch zur Herstellung von symmetrisch substituierten Onium-Salzen geeignet.
Das erfindungsgemäße Verfahren ist ebenfalls zur Herstellung von Onium-Salzen mit Diarylphosphinat oder gemischten Alkyl-aryl-phosphinaten geeignet. Aryl beschreibt hierbei insbesondere unsubstituiertes oder substituiertes Phenyl, wobei die Möglichkeiten der Substitution nachfolgend für Phenyl beschrieben wird und Alkyl eine für die Dialkylphosphinate beschriebene Bedeutung hat.

Die Aufgabe wird durch das erfindungsgemäße Verfahren gelöst, da der eingesetzte Ester das Anion des eingesetzten Oniumhalogenids alkyliert und nicht das organische Onium-Kation. Die als Nebenprodukt entstehenden Alkylhalogenide sind in der Regel Gase oder sehr flüchtige Verbindungen, die ohne großen prozesstechnischen Aufwand aus der Reaktionsmischung entfernt werden können. Einige dieser Nebenprodukte sind selbst wertvolle Materialien für organische Synthesen.

Gegenstand der Erfindung ist daher ein Verfahren zur Herstellung von Onium-Salzen mit Dialkylphosphinat-Anionen durch Umsetzung eines Onium-Halogenids mit einem Dialkylphosphinsäurealkylester oder einem Trialkylsilylester oder einem gemischten Alkyl-trialkylsilylester der Dialkylphosphinsäure nach Anspruch 1.

Geeignete Onium-Halogenide sind Phosphoniumhalogenide, Thiouroniumhalogenide, Guanidiniumhalogenide oder Halogenide mit heterocyclischem Kation, wobei die Halogenide aus der Gruppe Chloride, Bromide oder Iodide ausgewählt werden können. Bevorzugt werden im erfindungsgemäßen Verfahren Chloride oder Bromide eingesetzt.

Onium-Halogenide sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Phosphoniumhalogenide können beispielsweise durch die Formel (1)

[PR₄]⁺ Hal⁻ (1),

beschrieben werden,
wobei
Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit -F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/oder Atomgruppierungen ausgewählt aus der Gruppe -O-, -S-, -S(O)- oder -SO₂- ersetzt sein können.

Ausgeschlossen sind jedoch Verbindungen der Formel (1), in denen alle vier oder drei Substituenten R vollständig mit Halogenen substituiert sind, beispielsweise Tris(trifluormethyl)methylphosphoniumchlorid, Tetra(trifluormethyl)phosphoniumchlorid oder Tetra(nonafluorbutyl)phosphoniumchlorid.

Thiouroniumhalogenide können beispielsweise durch die Formel (2)

[(R¹R²N)-C(=SR⁷)(NR³R⁴)]⁺ Hal⁻ (2)

und Guanidiniumhalogenide können beispielsweise durch die Formel (3)

[C(NR¹R²)(NR³R⁴)(NR⁵R⁶)]⁺ Hal⁻ (3),

beschrieben werden,
wobei
Hal Cl, Br oder I und
R¹ bis R⁷ jeweils unabhängig voneinander
Wasserstoff oder CN, wobei Wasserstoff für R⁷ ausgeschlossen wird, geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit -F substituiert sein können, wobei jedoch nicht alle Substituenten an einem N-Atom vollständig mit F substituiert sein dürfen,
wobei die Substituenten R¹ bis R⁷ paarweise durch Einfach- oder Doppelbindung miteinander verbunden sein können
und wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -S-, -S(O)- oder -SO₂- ersetzt sein können.

Erfindungsgemäß zu verwendende Halogenide mit heterocyclischem Kation werden durch die Formel (4)

[HetN]⁺ Hal⁻ (4)

beschrieben, wobei
Hal Cl, Br oder I und
HetN⁺ ein heterocyclisches Kation, ausgewählt aus der Gruppe 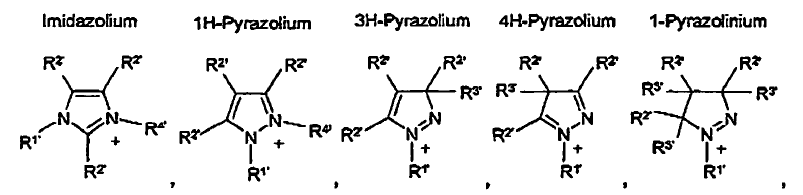 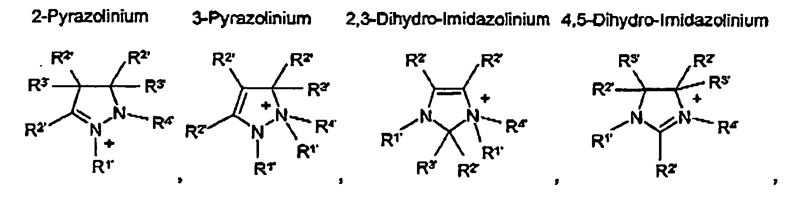 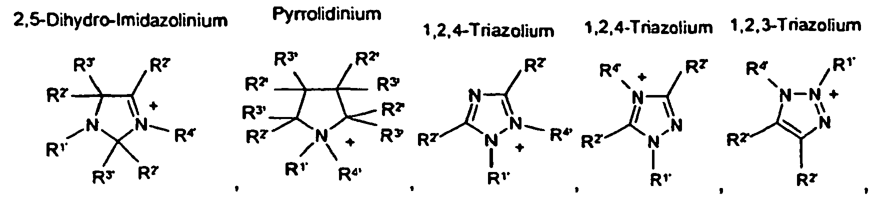 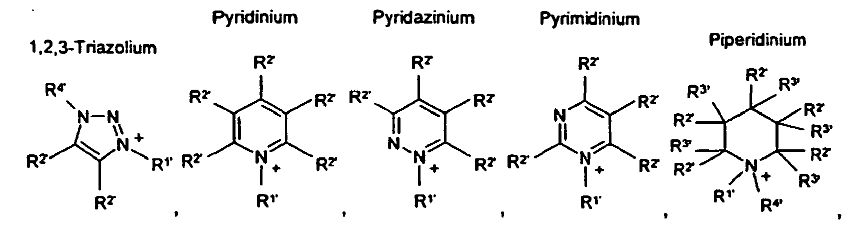 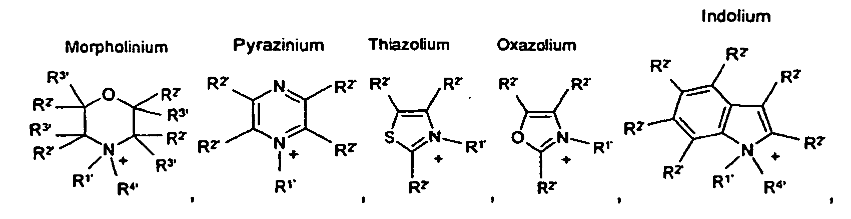 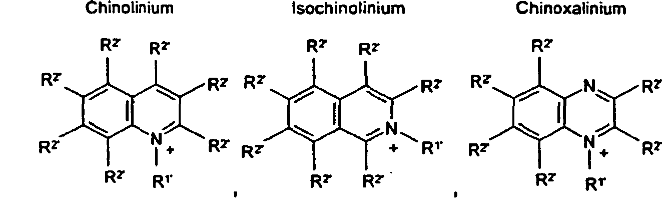 oder 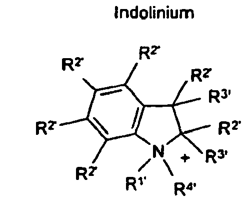 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R⁴' teilweise oder vollständig mit F substituiert sein können, wobei jedoch nicht gleichzeitig R^{1'} und R^{4'} CN sind oder vollständig mit F substituiert sein dürfen,
wobei die Substituenten R^{2'} und R^{3'} teilweise oder vollständig mit Halogenen oder teilweise mit NO₂ oder CN substituiert sein dürfen
und wobei ein oder zwei nicht benachbarte, nicht direkt am Heteroatom gebundene und nicht ω-ständige Kohlenstoffatome der Substituenten R¹' bis R⁴', durch Atome und/oder Atomgruppierungen ausgewählt aus der Gruppe -O-, -S-, -S(O)- oder -SO₂- ersetzt sein können.

Unter vollständig ungesättigten Substituenten werden im Sinne der vorliegenden Erfindung auch aromatische Substituenten verstanden.

Als Substituenten R und R¹ bis R⁷ der Verbindungen der Formeln (1) bis (3) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.
Die Substituenten R und R¹ bis R⁷ können jedoch ebenfalls durch weitere funktionelle Gruppen substituiert sein, beispielsweise durch CN, SO₂R', SO₂OR' oder COOR'. R' bedeutet nicht oder teilweise fluoriertes C₁- bis C₆ Alkyl, C₃- bis C₇-Cycloalkyl, unsubstituiertes oder substituiertes Phenyl.

Die Substituenten R in den Verbindungen der Formel (1) können dabei gleich oder verschieden sein. Bevorzugt sind drei Substituenten in Formel (1) gleich und ein Substituent verschieden.

Der Substituent R ist insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations [C(NR¹R²)(NR³R⁴)(NR⁵R⁶)]⁺ können auch paarweise derart verbunden sein, dass mono-, bi- oder polycyclische Kationen entstehen.

Ohne Einschränkung der Allgemeinheit sind Beispiele für solche Guanidinium-Kationen: 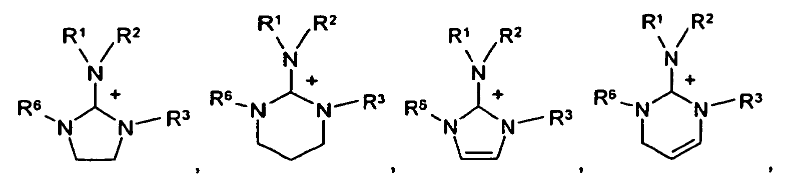 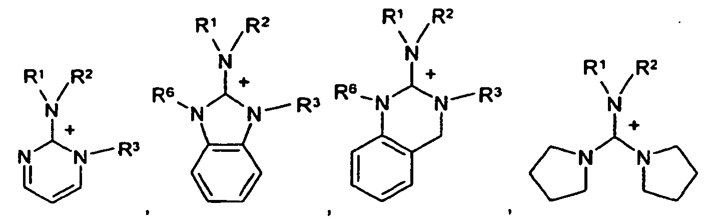 oder 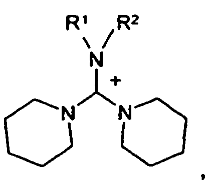 , wobei die Substituenten R¹ bis R³ und R⁶ eine zuvor angegebene oder besonders bevorzugte Bedeutung haben können.

Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R" oder substituiertes oder unsubstituiertes Phenyl substituiert sein, wobei X' und R" eine zuvor oder später angegebene Bedeutung haben.

Bis zu vier Substituenten des Thiouroniumkations [(R¹R²N)-C(=SR⁷)(NR³R⁴)]⁺ können auch paarweise derart verbunden sein, dass mono-, bi- oder polycyclische Kationen entstehen.
Ohne Einschränkung der Allgemeinheit sind Beispiele für solche Kationen im folgenden angegeben: 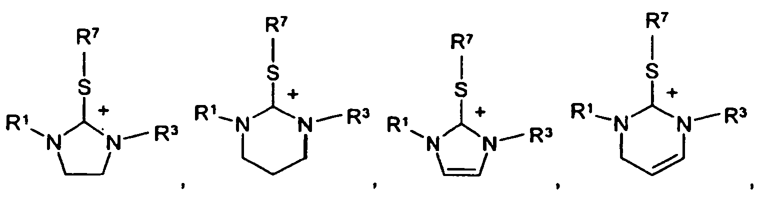 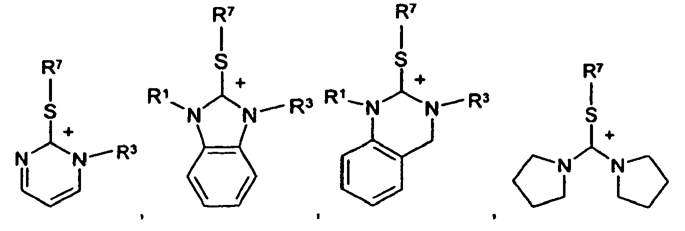 oder 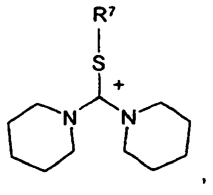 wobei die Substituenten R¹, R³ und R⁷ eine zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R" oder substituiertes oder unsubstituiertes Phenyl substituiert sein, wobei X' und R" eine zuvor oder später angegebene Bedeutung haben.

Die C₁-C₁₄-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluoriert, beispielsweise als Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -NO₂, substituiert sein können, besonders bevorzugt Benzyl oder Phenylpropyl. Der Phenylring oder auch die Alkylenkette können jedoch ebenfalls durch weitere funktionelle Gruppen substituiert sein, beispielsweise durch CN, SO₂R', SO₂OR' oder COOR', mit R' = nicht oder teilweise fluoriertes C₁- bis C₆-Alkyl, C₃- bis C₇-Cycloalkyl, unsubstituiertes oder substituiertes Phenyl.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder NO₂ substituiert sein kann.
Die Cycloalkylgruppen können jedoch ebenfalls durch weitere funktionelle Gruppen substituiert sein, beispielsweise durch CN, SO₂R', SO₂OR' oder COOR'. R' hat dabei eine zuvor definierte Bedeutung.

In den Substituenten R, R¹ bis R⁶ oder R^{1'} bis R^{4'} können auch ein oder zwei nicht benachbarte und nicht α-ständig zum Heteroatom oder ω gebundene gebundene Kohlenstoffatome, durch Atome und/oder Atomgruppierungen ausgewählt aus der Gruppe -O-, -S-, -S(O)- oder -SO₂- ersetzt werden.

Ohne Einschränkung der Allgemeinheit sind Beispiele für derart modifizierte Substituenten R, R¹ bis R⁶ und R^{1'} bis R^{4'}:
-OCH₃, -OCH(CH₃)₂, -CH₂OCH₃, -CH₂-CH₂-O-CH₃, -C₂H₄OCH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -CF₂CF₂H, -CF₂CHFCF₃, -CF₂CH(CF₃)₂, -C₂F₄N(C₂F₅)C₂F₅, -CHF₂, -CH₂CF₃, -C₂F₂H₃, -C₃FH₆, -CH₂C₃F₇, -CH₂C(O)OCH₃, -CH₂C₆H₅ oder -C(O)C₆H₅.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, C₁-C₆-Alkoxy, SCF₃, SO₂CF₃, COOR", SO₂X', SO₂NR"₂ oder SO₃R" substituiertes Phenyl, wobei X' F, Cl oder Br und R" ein nicht oder teilweise fluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-lodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (2) und (3) können dabei gleich oder verschieden sein. Besonders bevorzugt sind R¹ bis R⁷ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (4) kommen erfindungsgemäß dabei neben Wasserstoff bevorzugt in Frage: C₁- bis C₂₀₋, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl oder Aryl-C₁-C₆-alkyl.

Die Substituenten R^{1'} und R^{4'} sind jeweils unabhängig voneinander insbesondere bevorzugt Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt Methyl, Ethyl, n-Butyl oder Hexyl. In Pyrrolidinium-, Piperidinium oder Indolinium-Verbindungen sind die beiden Substituenten R^{1'} und R^{4'} bevorzugt unterschiedlich.

Der Substituent R^{2'} oder R^{3'} ist jeweils unabhängig voneinander insbesondere Wasserstoff, Methyl, Ethyl, Isopropyl, Propyl, Butyl, sek.-Butyl, tert.-Butyl, Cyclohexyl, Phenyl oder Benzyl. Besonders bevorzugt ist R^{2'} Wasserstoff, Methyl, Ethyl, Isopropyl, Propyl, Butyl, sek.-Butyl oder tert.-Butyl. Ganz besonders bevorzugt sind R^{2'} und R^{3'} Wasserstoff oder Methyl.

Bevorzugt sind die Alkylgruppen als Substituenten R^{1'} und R^{4'} der heterocyclischen Kationen der Formel (4) unterschiedlich von der Alkylgruppe des eingesetzten entsprechenden Esters, Trialkylsilylesters oder des gemischten Alkyl-trialkylsilylesters der Dialkylphosphinsäure. Das erfindungsgemäß hergestellte Onium-Dialkylphosphinat kann jedoch auch Alkylgruppen im Kation haben, die zu der Alkylgruppe im Ester gleich sind, die jedoch erfindungsgemäß nicht durch Alkylierung eingeführt wurden. Der Focus liegt dann auf der einfachen Reaktionsführung und des besonders niedrigen Halogenid-Gehalts im Endprodukt.

HetN⁺ der Formel (4) ist bevorzugt 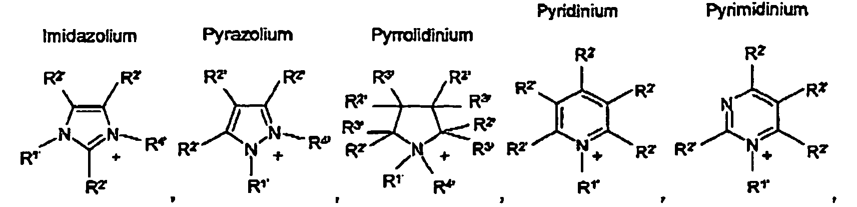 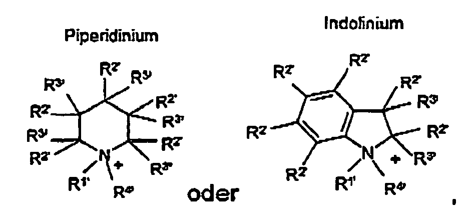 wobei die Substituenten R^{1'} bis R^{4'} jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben.

Als Ester einer Phosphinsäure wird bevorzugt ein entsprechender Ester mit geradkettigen oder verzweigten Alkylgruppen mit 1-8 C-Atomen, bevorzugt mit 1-4 C-Atomen eingesetzt, die jeweils unabhängig voneinander sind. Vorzugsweise sind die Alkylgruppen des Esters gleich.

Die eingesetzten Alkylester einer Phosphinsäure sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Beispiele für Dialkylphosphinsäureester sind Dimethylphosphinsäuremethylester, Dimethylphosphinsäureethylester, Bis(trifluormethyl)phosphinsäuremethylester, Diethylphosphinsäuremethylester, Diethylphosphinsäureethylester, Bis(pentafluorethyl)phosphinsäuremethylester, Bis(pentafluorethyl)phosphinsäureethylester oder Bis(nonafluorbutyl)phosphinsäuremethylester.

Als Trialkylsilylester der Dialkylphosphinsäure kann Trialkylsilyl-dialkylphosphinat, eingesetzt werden, wobei die Alkylgruppen linear oder verzweigt sein können mit 1 bis 8 C-Atomen, bevorzugt mit 1 bis 4 C-Atomen. Die Alkylgruppen der Trialkylsilylgruppe sind bevorzugt gleich und haben 1 bis 4 C-Atome.

Beispiele der zuvor genannten Ester sind
Trimethylsilyldimethylphosphinat, Triethylsilyldiethylphosphinat oder
Trimethylsilyl-bis(pentafluorethyl)phosphinat.

Die eingesetzten Trialkylsilylester oder gemischten Ester einer Phosphinsäure, wie zuvor beschrieben sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch
von an sich bekannten, hier nicht näher erwähnten Varianten Gebrauch machen.

Ein allgemeines Schema fasst das erfindungsgemäße Verfahren zusammen:

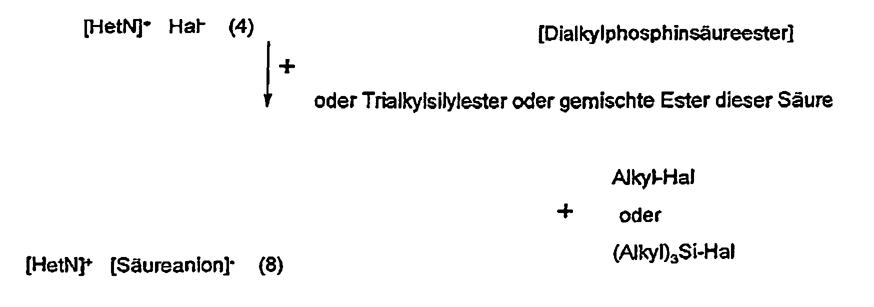

Die Substituenten HetN⁺ der Verbindungen der Formeln (4) und (8) entsprechen den Bedeutungen, wie zuvor beschrieben. [Säureanion]- bedeutet das entsprechende Anion aus dem eingesetzten Ester nach Abspaltung einer Alkylgruppe, beispielsweise [(Alkyl-O)₂P(O)O]⁻.

Die Reaktion wird erfindungsgemäß bei Temperaturen zwischen 20° und 100°C, bevorzugt bei 80° bis 100°, besonders bevorzugt bei 100°C durchgeführt, wenn Alkylester der entsprechenden Säuren eingesetzt werden. Werden die Trialkylsilylester oder die gemischten Ester der Säuren eingesetzt, findet die Reaktion zwischen 0°C und 30°C, bevorzugt bei Raumtemperatur statt. Es wird kein Lösungsmittel benötigt. Es können jedoch auch Lösungsmittel eingesetzt werden, beispielsweise Dimethoxyethan, Acetonitril, Aceton, Tetrahydrofuran, Dimethylformamid, Dimethylsulfoxid, Dioxan, Propionitril oder Gemische untereinander.

Die Umsetzung wird mit einem maximalen Überschuss bis 20% oder equimolarer Menge an dem entsprechenden Ester der Phosphor-, Phosphin- oder Phosphonsäure durchgeführt.

Die erfindungsgemäße Methode ist auch für die Reinigung von halogenidhaltigen Onium-Salzen mit Dialkylphosphinat-Anionen verwendbar.

Gegenstand der Erfindung sind auch die Ausgangsstoffe der Trialkylsilylester der Dialkylphosphinsäure, insbesondere (C₂F₅)₂P(O)OSi(Alkyl)₃, (C₃F₇)₂P(O)OSi(Alkyl)₃ und (C₄F₉)₂P(O)OSi(Alkyl)₃, wobei die Alkylgruppen der Trialkylsilylgruppe 1 bis 4 C-Atome haben kann. Bevorzugt sind die Alkylgruppen der Trialkylsilylgruppe gleich.
Besonders bevorzugte Trialkylsilylester sind
(C₂F₅)₂P(O)OSi(CH₃)₃, (C₂F₅)₂P(O)OSi(C₂H₅)₃, (C₃F₇)₂P(O)OSi(CH₃)₃, (C₃F₇)₂P(O)OSi(C₂H₅)₃, (C₄F₉)₂P(O)OSi(CH₃)₃ oder (C₄F₉)₂P(O)OSi(C₂H₅)₃.

Diese Verbindungen sind insbesondere hervorragende Silylierungsreagenzien, unabhängig von dem erfindungsgemäßen Verfahren.
Ein bekannter Trialkylsilylester ist (CF₃)₂P(O)OSi(CH₃)₃, wobei diese Verbindung jedoch schwer herstellbar und nicht stabil ist, da die F₃C-P-Bindung labil ist.

Auch ohne weitere Ausführungen wird davon ausgegangen, dass ein Fachmann die obige Beschreibung im weitesten Umfang nutzen kann.

Es versteht sich für den Fachmann von selbst, dass in den vorab und nachfolgend genannten Verbindungen Substituenten wie beispielsweise H, N, O, Cl, F durch die entsprechenden Isotope ersetzt sein können.

Die NMR-Spektren wurden an Lösungen in deuterierten Lösungsmitteln bei 20°C an einem Bruker ARX 400 Spektrometer mit einem 5 mm Breitbandkopf ¹H/BB mit Deuterium Lock gemessen, falls nicht in den Beispielen angegeben. Die Messfrequenzen der verschiedenen Kerne sind: ¹H: 400,13 MHz und ¹⁹F: 376,50 MHz. ³¹P-Spektren wurden an einem Bruker Avance 250 Spektromether gemessen mit der Meßfrequenz 101,26 MHz. Die Methode der Referenzierung wird bei jedem Spektrum bzw. bei jedem Datensatz separat angegeben.

### Beispiel 1:

### Synthese von 1-Butyl-3-methylimidazolium Bis(pentafluorethyl)phosphinat

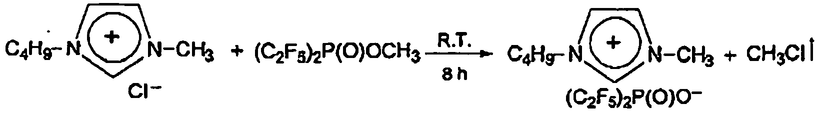

Eine Mischung von 0.693 g (3.97 mmol) 1-Butyl-3-methylimidazoliumchlorid und 1.256 g (3.97 mmol) Bis(Pentafluorethyl)phosphinsäuremethylester wird für 8 Stunden bei Raumtemperatur gerührt. NMR-Messungen bestätigen die Vollständigkeit der Reaktion. Der Rückstand wird für 30 Minuten bei 90°C im Vakuum von 13.3 Pa getrocknet. Man erhält 1.74 g 1-Butyl-3-methylimidazolium Bis(pentafluorethyl)phosphinat als Flüssigkeit.
Die Ausbeute ist annähernd quantitativ.

¹H NMR (Referenz: TMS; CD₃CN), ppm: 0.93 t (CH₃); 1.32 m (CH₂); 1.81 m (CH₂); 3.84 s (CH₃); 4.15 t (CH₂); 7.40 m (CH); 7.45 m (CH); 8.84 br. s. (CH); ³J_{H,H} = 7.4 Hz; ³J_{H,H} = 7.3 Hz.
¹⁹F NMR (Referenz: CCl₃F - intern; CD₃CN), ppm: -80.2 s (2CF₃); -124.9 d (2CF₂); ²J_{P,F} = 66 Hz.
³¹P NMR (Referenz: 85% H₃PO₄ - extern; CD₃CN), ppm: -2.5 quin.; ²J_{P,F} = 66 Hz.

### Beispiel 2:

### Synthese von 1-Butyl-3-methylimidazolium Bis(pentafluorethyl)phosphinat

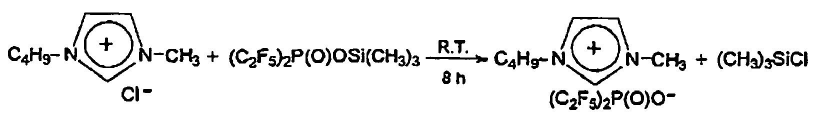

Eine Mischung von 0.506 g (2.30 mmol) 1-Butyl-3-methylimidazoliumchlorid und 1.084 g (2.30 mmol)
Bis(pentafluorethyl)phosphinsäuretrimethylsilylester wird 8 Stunden bei Raumtemperatur gerührt. NMR-Messungen zeigen die Vollständigkeit der Reaktion. Der Rückstand wird 30 Minuten bei 90°C und 13.3 Pa getrocknet. Man erhält 1.275 g 1-Butyl-3-methylimidazolium Bis(pentafluorethyl)phosphinat als Flüssigkeit. Die Ausbeute ist annähernd quantitativ.
Die NMR-Spektren entsprechen Beispiel 1.

### Beispiel 3:

### Synthese von 1-Butylpyridinium Bis(pentafluorethyl)phosphinat

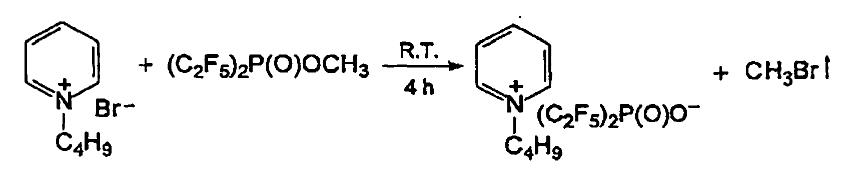

Eine Mischung von 0.83 g (3.84 mmol) 1-Butyl-pyridiniumbromid und 1.22 g (3.86 mmol) Bis(Pentafluorethyl)phosphinsäuremethylester wird für 4 Stunden bei Raumtemperatur gerührt. NMR-Messungen bestätigen die Vollständigkeit der Reaktion. Der Rückstand wird für 30 Minuten bei 90°C im Vakuum von 13.3 Pa getrocknet. Man erhält 1.67 g 1-Butylpyridinium Bis(pentafluorethyl)phosphinat als Flüssigkeit. Die Ausbeute ist annähernd quantitativ.

¹H NMR (Referenz: TMS; CD₃CN), ppm: 0.95 t (CH₃); 1.37 m (CH₂); 1.95 m (CH₂); 4.56 t (CH₂); 8.03 m (2CH); 8.52 t,t (CH); 8.83 d (2CH); ³J_{H,H} = 7.3 Hz; ³J_{H,H} = 7.5 Hz; ³J_{H,H} = 7.8 Hz; ³J_{H,H} = 5.7 Hz; ⁴_{H,H} = 1.2 Hz.
¹⁹F NMR (Referenz: CCl₃F - intern; CD₃CN), ppm: -80.2 s (2CF₃); -124.9 d (2CF₂); ²J_{P,F} = 66 Hz.
³¹P NMR (Referenz: 85% H₃PO₄ - extern; CD₃CN), ppm: -2.5 quin.; ²J_{P,F} = 66 Hz.

## Patentansprüche

1. Verfahren zur Herstellung von heterocyclischen Onium-Salzen mit Dialkylphosphinat-Anionen durch Umsetzung eines Onium-Halogenids der Formel (4)
[HetN]⁺Hal⁻ (4),
wobei
Hal Cl, Br oder I und
HetN⁺ ein heterocyclisches Kation, ausgewählt aus der Gruppe 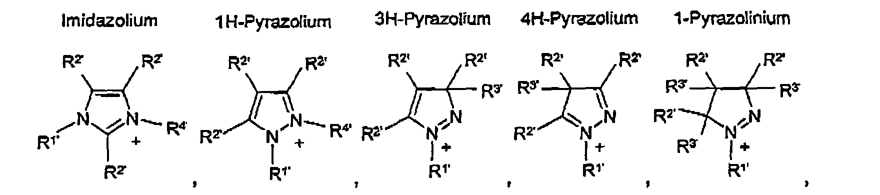 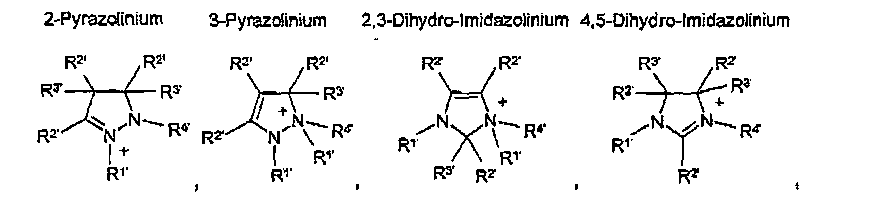 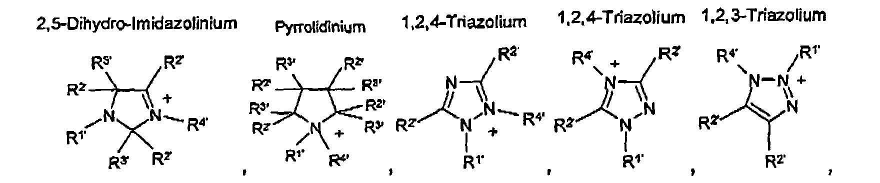 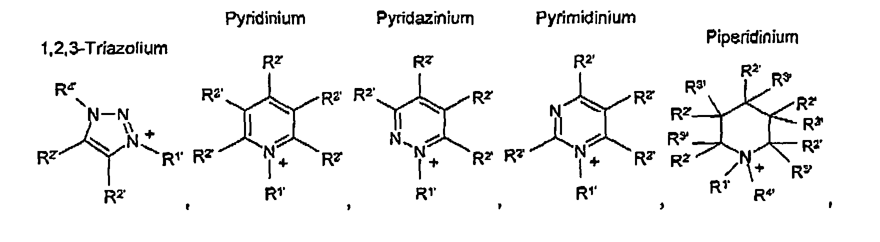 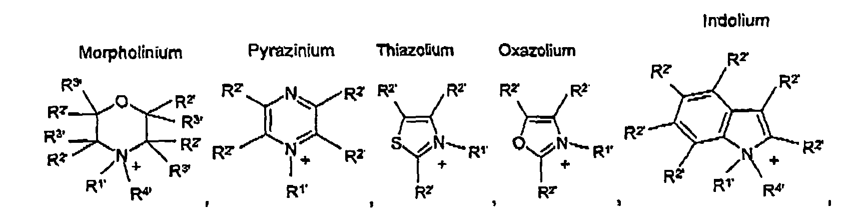 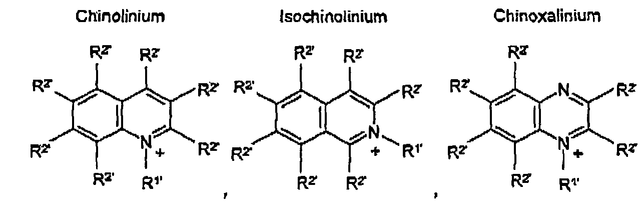 oder 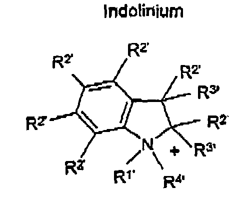 bedeutet, wobei die Substituenten
R^{1'} bis R^{4'} jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt, gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R^{4'} teilweise oder vollständig mit F substituiert sein können, wobei jedoch nicht gleichzeitig R^{1'} und R^{4'} CN sind oder vollständig mit F substituiert sein dürfen,
wobei die Substituenten R^{2'} und R^{3'} teilweise oder vollständig mit Halogenen oder teilweise mit NO₂ oder CN substituiert sein dürfen und wobei ein oder zwei nicht benachbarte, nicht direkt am Heteroatom gebundene und nicht ω-ständige Kohlenstoffatome der Substituenten R^{1'} bis R^{4'}, durch Atome und/oder Atomgruppierungen ausgewählt aus der Gruppe -O-, -S-, -S(O)- oder -SO₂- ersetzt sein können,
mit einem Dialkylphosphinsäurealkylester oder einem Trialkylsilylester oder einem gemischten Alkyl-trialkylsilylester der Dialkylphosphinsäure.

2. Verfahren nach Anspruch 1, **dadurch gekennzeichnet, dass** die Reaktion der Alkylester der Dialkylphosphin- säure bei Temperaturen von 20°C bis 100°C durchgeführt wird.

3. Verfahren nach Anspruch 1, **dadurch gekennzeichnet, dass** die Reaktion der Trialkylsilylester der Dialkylphosphin-säure bei Temperaturen von 0°C bis 30°C durchgeführt wird.

4. Verfahren nach einem oder mehreren der Ansprüche 1 bis 3, **dadurch gekennzeichnet, dass** die Reaktion ohne Lösungsmittel durchgeführt wird.

5. Verwendung des Verfahrens nach einem oder mehreren der Ansprüche 1 bis 4 zur Aufreinigung von durch Onium-Halogenide verunreinigten ionischen Flüssigkeiten mit Dialkylphosphinat-Anionen.

6. Trialkylsilylester der Formeln (C₂F₅)₂P(O)OSi(Alkyl)₃, (C₃F₇)₂P(O)OSi(Alkyl)₃ oder (C₄F₉)₂P(O)OSi(Alkyl)₃, wobei die Alkylgruppen der Trialkylsilylgruppe 1 bis 4 C-Atome haben kann.

7. Trialkylsilylester nach Anspruch 6, entsprechend der Verbindungen (C₂F₅)₂P(O)OSi(CH₃)₃, (C₂F₅)₂P(O)OSi(C₂H₅)₃, (C₃F₇)₂P(O)OSi(CH₃)₃, (C₃F₇)₂P(O)OSi(C₂H₅)₃, (C₄F₉)₂P(O)OSi(CH₃)₃ oder (C₄F₉)₂P(O)OSi(C₂H₅)₃.

## Claims

1. Process for the preparation of heterocyclic onium salts with dialkylphosphinate anions by reaction of an onium halide of the formula (4)
[HetN]⁺Hal⁻ (4),
where
Hal denotes Cl, Br or I and
HetN⁺ denotes a heterocyclic cation selected from the group 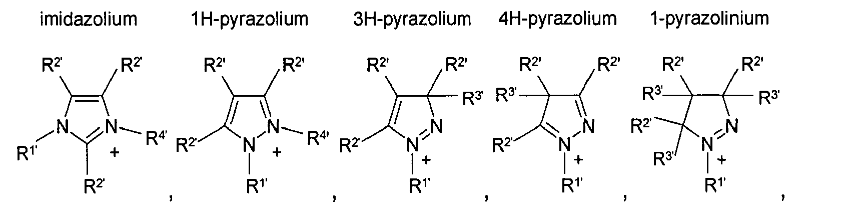 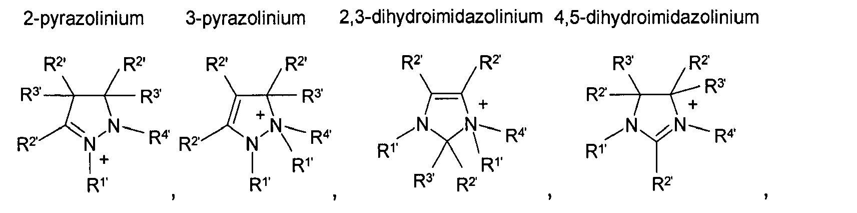 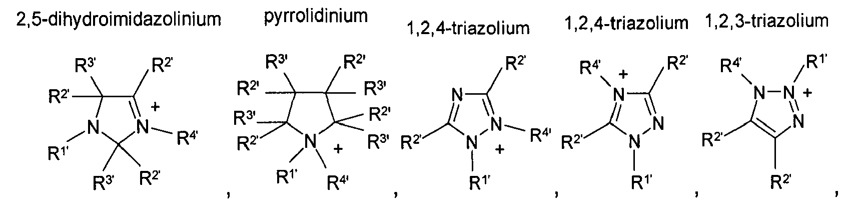 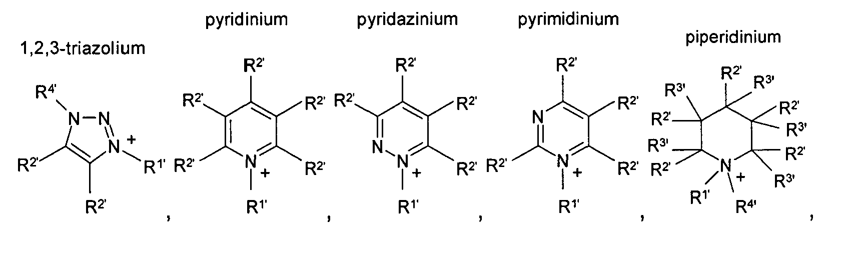 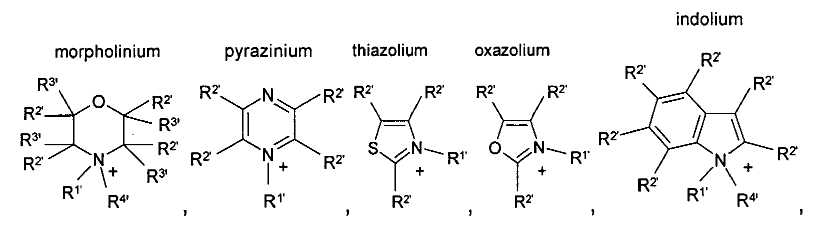 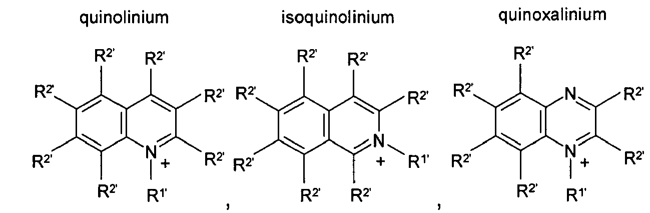 or 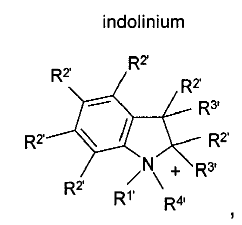 where the substituents
R^{1'} to R^{4'} each, independently of one another, denote hydrogen or CN,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino containing alkyl groups having 1-4 C atoms, but which is not bonded to the heteroatom of the heterocycle,
saturated, partially or fully unsaturated cycloalkyl having 3-7 C atoms, which may be substituted by alkyl groups having 1-6 C atoms,
or aryl-C₁-C₆-alkyl,
where the substituents R^{1'} and R^{4'} may be partially or fully substituted by F, but where R^{1'} and R^{4'} are not simultaneously CN or cannot simultaneously be fully substituted by F,
where the substituents R^{2'} and R^{3'} may be partially or fully substituted by halogens or partially substituted by NO₂ or CN
and where one or two non-adjacent carbon atoms in the substituents R^{1'} to R^{4'} which are not bonded directly to the heteroatom and are not in the ω-position may be replaced by atoms and/or atom groups selected from the group -O-, -S-, -S(O)- or -SO₂-,
with an alkyl dialkylphosphinate or a trialkylsilyl ester or a mixed alkyl trialkylsilyl ester of dialkylphosphinic acid.

2. Process according to Claim 1, **characterised in that** the reaction of the alkyl esters of dialkylphosphinic acid is carried out at temperatures of 20°C to 100°C.

3. Process according to Claim 1, **characterised in that** the reaction of the trialkylsilyl esters of dialkylphosphinic acid is carried out at temperatures of 0°C to 30°C.

4. Process according to one or more of Claims 1 to 3, **characterised in that** the reaction is carried out without solvents.

5. Use of the process according to one or more of Claims 1 to 4 for the purification of ionic liquids with dialkylphosphinate anions which are contaminated by onium halides.

6. Trialkylsilyl esters of the formulae (C₂F₅)₂P(O)OSi(alkyl)₃, (C₃F₇)₂P(O)OSi(alkyl)₃ or (C₄F₉)₂P(O)OSi(alkyl)₃, where the alkyl groups of the trialkylsilyl group can have 1 to 4 C atoms.

7. Trialkylsilyl esters according to Claim 6, corresponding to the compounds (C₂F₅)₂P(O)OSi(CH₃)₃, (C₂F₅)₂P(O)OSi(C₂H₅)₃, (C₃F₇)₂P(O)OSi(CH₃)₃, (C₃F₇)₂P(O)OSi(C₂H₅)₃, (C₄F₉)₂P(O)OSi(CH₃)₃ or (C₄F₉)₂P(O)OSi(C₂H₅)₃.

## Revendications

1. Procédé pour la préparation de sels d'onium hétérocycliques avec des anions dialkylphosphinate par réaction d'un halogénure d'onium de la formule (4)
[HetN]⁺Hal⁻ (4),
dans laquelle
Hal représente Cl, Br ou I est
HetN⁺ représente un cation hétérocyclique choisi parmi le groupe 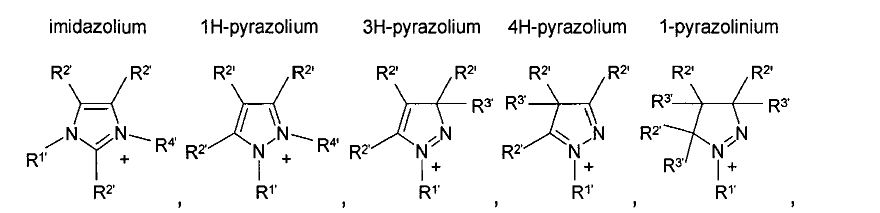 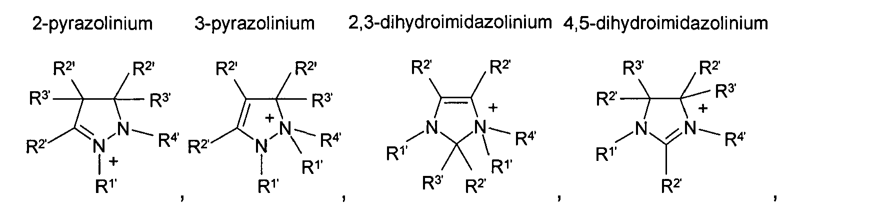 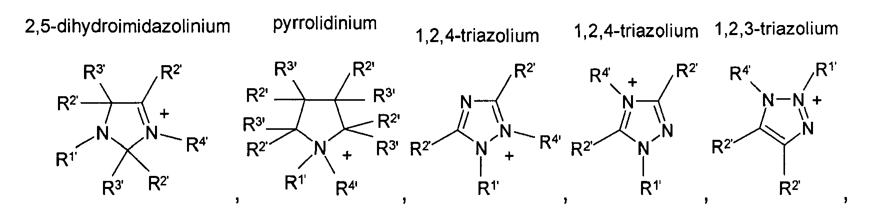 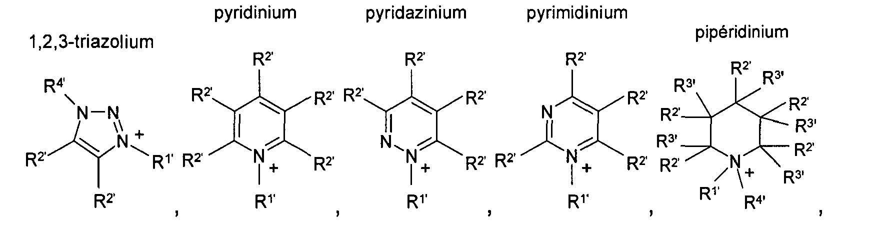 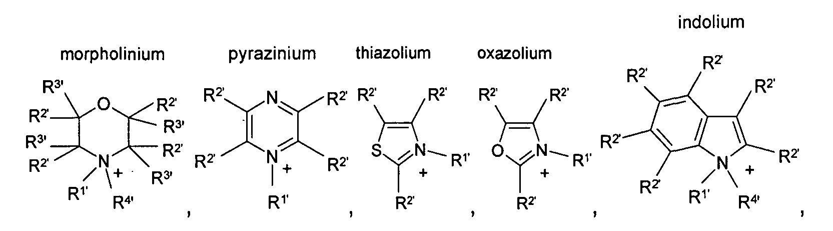 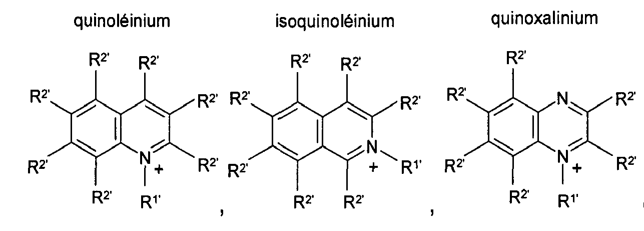 ou 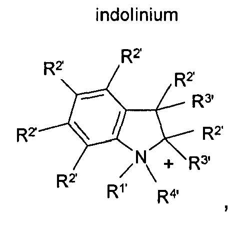 dans lesquels les substituants
R^{1'} à R^{4'} chacun, indépendamment des autres, représentent hydrogène ou CN,
alkyle en chaîne droite ou ramifié ayant 1-20 atomes de C,
alkényle en chaîne droite ou ramifié ayant 2-20 atomes de C et une ou plusieurs doubles liaisons,
alkynyle en chaîne droite ou ramifié ayant 2-20 atomes de C et une ou plusieurs triples liaisons,
dialkylamino contenant des groupes alkyle ayant 1-4 atomes de C, mais qui n'est pas lié à l'hétéroatome de l'hétérocycle,
cycloalkyle saturé, partiellement ou complètement non saturé ayant 3-7 atomes de C, qui peut être substitué par des groupes alkyle ayant 1-6 atomes de C,
ou aryl-C₁-C₆-alkyl,
dans lesquels les substituants R^{1'} et R^{4'} peuvent être partiellement ou complètement substitués par F, mais dans lesquels R^{1'} et R^{4'} ne sont pas simultanément CN ou ne peuvent pas simultanément être complètement substitués par F,
dans lesquels les substituants R^{2'} et R^{3'} peuvent être partiellement ou complètement substitués par halogènes ou partiellement substitués par NO₂ ou CN
et dans lesquels un ou deux atomes de carbone non adjacents dans les substituants R^{1'} à R^{4'} qui ne sont pas liés directement à l'hétéroatome et qui ne sont pas dans la position ω peuvent être remplacés par des atomes et/ou des groupes d'atomes choisis parmi le groupe -O-, -S-, -S(O)- ou -SO₂-,
avec un dialkylphosphinate d'alkyle ou un ester de trialkylsilyle ou un ester d'alkyle trialkylsilyle mélangé d'acide dialkylphosphinique.

2. Procédé selon la revendication 1, **caractérisé en ce que** la réaction des esters d'alkyle d'acide dialkylphosphinique est effectuée à des températures de 20°C à 100°C.

3. Procédé selon la revendication 1, **caractérisé en ce que** la réaction des esters de trialkylsilyle esters d'acide dialkylphosphinique est effectuée à des températures de 0°C à 30°C.

4. Procédé selon une ou plusieurs des revendications 1 à 3, **caractérisé en ce que** la réaction est effectuée sans solvant.

5. Utilisation du procédé selon une ou plusieurs des revendications 1 à 4 pour la purification de liquides ioniques avec des anions de dialkylphosphinate qui sont contaminés par des halogénures d'onium.

6. Esters de trialkylsilyle des formules (C₂F₅)₂P(O)OSi(alkyl)₃, (C₃F₇)₂P(O)OSi(alkyl)₃ ou (C₄F₉)₂P(O)OSi(alkyl)₃, dans lesquels les groupes alkyle du groupe trialkylsilyle peuvent avoir 1 à 4 atomes de C.

7. Esters de trialkylsilyle selon la revendication 6, correspondant aux composés (C₂F₅)₂P(O)OSi(CH₃)₃, (C₂F₅)₂P(O)OSi(C₂H₅)₃, (C₃F₇)₂P(O)OSi(CH₃)₃, (C₃F₇)₂P(O)OSi(C₂H₅)₃, (C₄F₉)₂P(O)OSi(CH₃)₃ OU (C₄F₉)₂P(O)OSi(C₂H₅)₃.
